# EUROPÄISCHE PATENTSCHRIFT

(11) **EP 1 830 727 B1**
(45) Veröffentlichungstag und Bekanntmachung des Hinweises auf die Patenterteilung: **29.08.2012**
(21) Anmeldenummer: 04802398.0
(22) Anmeldetag: 31.12.2004
(51) Int. Cl.: A61B 17/72, A61B 17/86

(54) **MARKNAGEL**
INTRAMEDULLARY NAIL
CLOU MEDULLAIRE

(43) Veröffentlichungstag der Anmeldung: 12.09.2007
(73) Patentinhaber: Synthes GmbH, 4436 Oberdorf (CH)
(72) Erfinder: BUETTLER, Markus, CH-4702 Oensingen (CH); SCHLIENGER, André, CH-4144 Arlesheim (CH); FRIGG, Robert, CH-2544 Bettlach (CH)
(74) Vertreter: Lusuardi, Werther
(86) Internationale Anmeldenummer: PCT/CH2004/000758
(87) Internationale Veröffentlichungsnummer: WO 2006/069453

(56) Entgegenhaltungen:
- EP-A- 0 827 717
- WO-A-00/71040
- CH-A5- 674 613
- US-A1- 2002 183 750
- US-A1- 2003 069 581

## Beschreibung

Die Erfindung bezieht sich auf einen Marknagel, insbesondere für die Tibia gemäss dem Oberbegriff des Patentanspruchs 1.

Aus der US 6,010,506 GOSNEY ist ein gattungsgemässer Marknagel bekannt, der ein aufpressbares proximales und ein distales Endteil aufweist, welche mittels mehrerer axial zwischen den Endteilen angeordneter, gekrümmter Segmente relativ zueinander abgewinkelt sind. Zudem ist das aufpressbare proximale Endteil abgekröpft, so dass zwischen den an den axialen Enden an die Zentralachsen angelegten Tangenten ein relativ grosser Winkel gebildet wird. Nachteilig an diesem bekannten Marknagel ist, dass er für Anwendungen in relativ geradlinigen Röhrenknochen, insbesondere der Tibia nicht geeignet ist. Der Obergriff des Anspruchs 1 basiert of dieser Offenbarung.

CH 674 613, offenbart einen Marknagel, insbesondere für die Tibia, mit einem proximalen Endteil, einem distalen zur Einführung in den Markraum geeigneten Endteil und einer Zentralachse , der Unterschied zu der CH 674 613 und Anspruch 1 ist das Merkmal D, d.h. die Tangenten in den beiden Endpunkten eines die beiden gekrümmten Abschnitte umfassenden, mittleren Abschnittes einen Winkel Gamma zwischen 9° und 12°einschliessen.

Hier will die Erfindung Abhilfe schaffen. Der Erfindung liegt die Aufgabe zugrunde, einen Marknagel zu schaffen, welcher einen mehrere gekrümmte Abschnitte umfassenden, mittleren Abschnitt aufweist, wobei die Tangenten in den beiden Endpunkten dieses mittleren Abschnittes einen relativ kleinen, d.h. insbesondere für geradlinige Röhrenknochen geeigneten Winkel einschliessen.

Die Erfindung löst die gestellte Aufgabe mit einem Marknagel, welcher die Merkmale des Anspruchs 1 aufweist.

Die durch die Erfindung erreichten Vorteile sind im wesentlichen darin zu sehen, dass dank des erfindungsgemässen Marknagels:
- der Marknagel einen zwei gekrümmte Abschnitte umfassende, mittleren Abschnitt aufweist, und wegen der zwei, einen unterschiedlichen Radius aufweisenden Krümmungen eine optimale Anpassung des Marknagels an den Markkanal erreichbar ist;
- die Tangenten in den Endpunkten dieses mittleren Abschnitts einen Winkel gamma zwischen 9° und 12° einschliessen und der Marknagel daher für relativ geradlinige Röhrenknochen, insbesondere die Tibia geeignet ist; und
- eine Insertion des Marknagels in den Markraum wegen des kleinen Winkels gamma ohne grossen Kraftaufwand möglich ist.

Je nach Anwendung weist der Marknagel in verschiedenen Ausführungsformen die folgenden Merkmale auf:
- die Länge G₃ des proximalen Endteils liegt im Bereich von 1/6 L bis 1/3 L, wobei L die Gesamtlänge des Marknagels ist;
- die Tangenten in den beiden Endpunkten des ersten gekrümmten Abschnitts schliessen einen Winkel alpha im Bereich von 7° -10 °, vorzugsweise 8° - 9° ein;
- die Tangenten in den beiden Endpunkten des zweiten gekrümmten Abschnitts schliessen einen Winkel beta im Bereich von 1° - 3° ein;
- der erste gekrümmte Abschnitt der Länge G₁ weist einen Krümmungsradius R₁ im Bereich von 300 - 1300 mm auf;
- das Verhältnis L/R₁ liegt im Bereich von 0,2 bis 0,8;
- die Länge "I" des geraden distalen Abschnittes liegt im Bereich von 0,20 L bis 0,55 L, vorzugsweise im Bereich von 0,25 L bis 0,50 L;
- die Länge G₁ des ersten gekrümmten Abschnitts liegt im Bereich von 0,2 bis 0,4 L; und
- der Krümmungsradius R₂ des zweiten gekrümmten Abschnitts liegt im Bereich von 10 bis 50 mm.

In einer weiteren Ausführungsform grenzt das proximale Endteil axial an den zweiten gekrümmten Abschnitt an und als gerader proximaler Abschnitt ausgebildet.

In wiederum einer weiteren Ausführungsform ist das distale Endteil als gerader Abschnitt ausgebildet.

Durch die Ausbildung eines oder beider Endteile als gerade Abschnitte ist der Vorteil erreichbar, dass die Krümmung des Marknagels gering ist, wodurch der Marknagel insbesondere für gerade Röhrenknochen geeignet ist.

In einer anderen Ausführungsform weist der Marknagel eine zur Zentralachse koaxiale Längsbohrung auf.

In wiederum einer anderen Ausführungsform ist im Bereich des proximalen Endteils mindestens ein quer zur Zentralachse verlaufendes Verriegelungsloch vorhanden, welches zur Aufnahme einer Schraube geeignet ist.

In einer weiteren Ausführungsform ist im Bereich des distalen Endteils mindestens ein quer zur Zentralachse verlaufendes Verriegelungsloch vorhanden, welches ebenfalls zur Aufnahme einer Schraube geeignet ist.

In wiederum einer weiteren Ausführungsform sind im Bereich des distalen Endteils zwei quer zur Zentralachse verlaufende Verriegelungslöcher vorhanden, welche vorzugsweise untereinander einen Winkel von 45° bis 90° Grad einschliessen.

In einer anderen Ausführungsform weist das distale Endteil vier Verriegelungslöcher auf, wobei das proximal gelegene, mittlere Verriegelungsloch zu den beiden anderen Verriegelungslöchern eine unterschiedlich Distanz aufweist.

Im folgenden wird das Implantationsverfahren anhand eines kannulierten Marknagels kurz beschrieben:
Schritt A: Erstellen und Halten der optimalen Reposition abhängig vom Frakturtyp
Schritt B: Eröffnung des Markkanals mit Hilfe eines Eröffnungsinstrumentariums, so dass der Eintrittswinkel und die Orientierung zum Markkanal - gemäss der verwendeten Operationstechnik - stimmt.
Schritt C: Einführung eines Führungsdrahtes bis in die distale, zukünftige Endlage des Marknagels und Bestimmung der Länge des erforderlichen Marknagels;
Schritt D: Der auf dem Insertionshandgriff vormontierte Markangel wird über dem Führungsdraht durch den Eröffnungskanal in den Markraum eingebracht
Schritt E: Nach Kontrolle der axialen Lage des Marknagels und der Reposition wird der Marknagel über seine Verriegelungsoptionen verriegelt.

Die Erfindung und Weiterbildungen der Erfindung werden im folgenden anhand der teilweise schematischen Darstellungen eines Ausführungsbeispiels noch näher erläutert.

Es zeigen:
Fig. 1 einen Längsschnitt durch eine Ausführungsform des erfindungsgemässen Marknagels;
Fig. 2 einen Schnitt längs der Linie II-II in Fig. 1;
Fig. 3 einen vergrösserten Ausschnitt im Bereich des zweiten gekrümmten Abschnittes der in den Fig. 1 und 2 dargestellten Ausführungsform des erfindungsgemässen Marknagels.

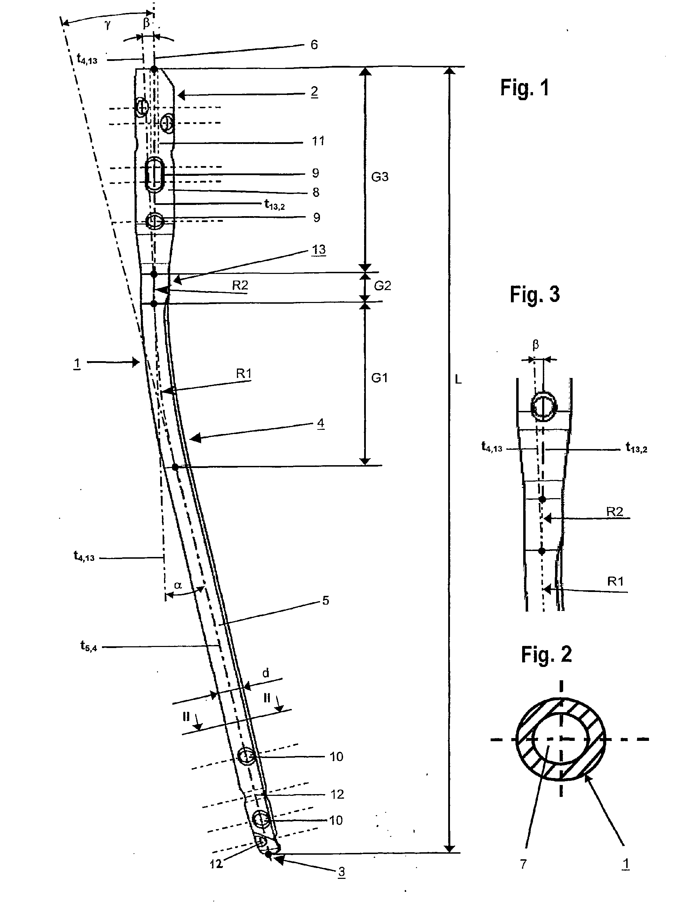

Der in den Fig. 1 bis 3 dargestellte Marknagel 1 umfasst im wesentlichen eine Zentralachse 6, ein proximales Endteil 2, ein distales, zur Einführung in den Markraum geeignetes Endteil 3 und zwei axial hintereinander angeordnete gekrümmte Abschnitte 4;13. Das proximale Endteil 2 grenzt axial an den zweiten gekrümmten Abschnitt 13 an und ist als gerader proximaler Abschnitt 8 der Länge G₃ ≤ L ausgebildet während das distale Endteil 3 als gerader distaler Abschnitt 5 mit der Länge "I" ausgebildet ist. Der erste gekrümmte Abschnitt 4 weist eine Länge G₁ ≤ L und einen Krümmungsradius R₁ auf, während der zweite gekrümmte Abschnitt 13 zwischen dem proximalen Endteil 2 und dem ersten gekrümmten Abschnitt 4 angeordnete, zweite gekrümmte Abschnitt 13 eine Länge G₂ ≤ G₁ und einen Krümmungsradius R₂ < R₁ aufweist. Die Tangenten t_{5,4} und t_{13,2} in den beiden Endpunkten eines die beiden gekrümmten Abschnitte 4;13 umfassenden, mittleren Abschnittes schliessen hier einen Winkel gamma von 10° ein und fallen proximal mit der Zentralachse 6 des geraden proximalen Abschnittes 8 und distal mit der Zentralachse 6 des geraden distalen Abschnittes 5 zusammen.

Ferner schliessen die Tangenten t_{5,4} und t_{4,13} in den beiden Endpunkten des ersten gekrümmten Abschnitts 4 hier einen Winkel alpha von 8° und die Tangenten t_{4,13} und t_{13,2} in den beiden Endpunkten des zweiten gekrümmten Abschnitts 13 einen Winkel beta von 2° ein.

Beide Krümmungen des Marknagels liegen in der Zeichenebene, welche - nach erfolgter Implantation des Marknagels 1 - der anatomischen medio-lateralen Ebene entspricht, d.h. der Marknagel 1 ist nach erfolgter Implantation in antero-posteriorer Richtung gebogen.

Im weiteren weist der Marknagel eine zur Zentralachse 6 koaxiale, durchgehende Längsbohrung 7 (Fig. 2) auf.

Im Bereich des proximalen Endteils 2 des Marknagels 1 sind vier quer zur Zentralachse 6 verlaufende Verriegelungslöcher 9 vorhanden, wobei eines davon als Langloch ausgebildet ist, um eine Kompression durchführen zu können. Für die Verriegelung des Marknagels 1 sind im Bereich des proximalen Endteils 2 fünf Schrauben (nicht gezeichnet) vorgesehen.

Im Bereich des distalen Endteils 3 sind vier quer zur Zentralachse 6 verlaufende Verriegelungslöcher 10,12,10,12 vorhanden, welche in unterschiedlichen radialen Richtungen angeordnet sind und zueinander unterschiedliche Winkel einschliessen. Dabei weist das mittlere Verriegelungsloch 12 zu den beiden anderen Verriegelungslöchern 10 eine unterschiedlich Distanz auf.

## Patentansprüche

1. Marknagel (1), insbesondere für die Tibia, mit einem proximalen Endteil (2), einem distalen zur Einführung in den Markraum geeigneten Endteil (3) und einer Zentralachse (6), der
A) eine Gesamtlänge L im Bereich von 200 bis 500 mm aufweist;
B) einen ersten gekrümmten Abschnitt (4) der Länge G₁ ≤ L und mit dem Krümmungsradius R₁ aufweist; und
C) einen - zwischen dem proximalen Endteil (2) und dem ersten gekrümmten Abschnitt (4) angeordneten - zweiten gekrümmten Abschnitt (13) der Länge G₂ ≤ G₁ und mit dem Krümmungsradius R₂ < R₁ aufweist;
**dadurch gekennzeichnet, dass**
D) die Tangenten in den beiden Endpunkten eines die beiden gekrümmten Abschnitte (4;13) umfassenden, mittleren Abschnittes einen Winkel gamma zwischen 9° und 12° einschliessen.

2. Marknagel (1) nach Anspruch 1, **dadurch gekennzeichnet, dass** die Länge G₃ des proximalen Endteils (2) im Bereich von 1/6 L bis 1/3 L liegt.

3. Marknagel (1) nach Anspruch 1 oder 2, **dadurch gekennzeichnet, dass** die Tangenten t_{5,4} und t_{4,13} in den beiden Endpunkten des ersten gekrümmten Abschnitts (4) einen Winkel alpha im Bereich von 7° - 10 °, vorzugsweise 8° - 9° einschliessen.

4. Marknagel (1) nach einem der Ansprüche 1 bis 3, **dadurch gekennzeichnet, dass** die Tangenten t_{4,13} und t_{13,2} in den beiden Endpunkten des zweiten gekrümmten Abschnitts (13) einen Winkel beta im Bereich von 1° - 3° einschliessen.

5. Marknagel (1) nach einem der Ansprüche 1 bis 4, **dadurch gekennzeichnet, dass** das proximale Endteil (2) axial an den zweiten gekrümmten Abschnitt (13) angrenzt und als gerader Abschnitt (8) der Länge G₃ ≤ L ausgebildet ist.

6. Marknagel (1) nach einem der Ansprüche 1 bis 5, **dadurch gekennzeichnet, dass** der erste gekrümmte Abschnitt (4) der Länge G₁ einen Krümmungsradius R₁ im Bereich von 300 -1300 mm aufweist.

7. Marknagel (1) nach einem der Ansprüche 1 bis 6, **dadurch gekennzeichnet, dass** das Verhältnis L/R₁ im Bereich von 0,2 bis 0,8 liegt.

8. Marknagel (1) nach einem der Ansprüche 1 bis 7, **dadurch gekennzeichnet, dass** das distale Endteil (3) als gerader Abschnitt (5) mit der Länge "I" ≤ L ausgebildet ist.

9. Marknagel (1) nach Anspruch 7, **dadurch gekennzeichnet, dass** die Länge "I" im Bereich von 0,20 L bis 0,55 L liegt.

10. Marknagel (1) nach Anspruch 8, **dadurch gekennzeichnet, dass** die Länge "I" im Bereich von 0,25 L bis 0,50 L liegt.

11. Marknagel (1) nach einem der Ansprüche 1 bis 10, **dadurch gekennzeichnet, dass** er zur Zentralachse (6) koaxiale Längsbohrung (7) aufweist

12. Marknagel (1) nach einem der Ansprüche 1 bis 11, **dadurch gekennzeichnet, dass** im Bereich des proximalen Endteils (2) mindestens ein quer zur Zentralachse (6) verlaufendes Verriegelungsloch (9) vorhanden ist.

13. Marknagel (1) nach einem der Ansprüche 1 bis 12, **dadurch gekennzeichnet, dass** im Bereich des distalen Endteils (3) mindestens ein quer zur Zentralachse (6) verlaufendes Verriegelungsloch (10) vorhanden ist

14. Marknagel (1) nach einem der Ansprüche 1 bis 13, **dadurch gekennzeichnet, dass** im Bereich des distalen Endteils (3) zwei quer zur Zentralachse (6) verlaufende Verriegelungslöcher (10) vorhanden sind.

15. Marknagel (1) nach Anspruch 14, **dadurch gekennzeichnet, dass** die beiden quer zur Zentralachse (6) verlaufenden Verriegelungslöcher (10) untereinander einen Winkel von 45° bis 90° Grad einschliessen.

16. Marknagel (1) nach einem der Ansprüche 1 bis 15, **dadurch gekennzeichnet, dass** das distale Endteil (3) drei Verriegelungslöcher (10,12,10) aufweist, wobei das mittlere Verriegelungsloch (12) zu den beiden anderen Verriegelungslöchern (10) eine unterschiedlich Distanz aufweist.

17. Marknagel (1) nach einem der Ansprüche 1 bis 16, **dadurch gekennzeichnet, dass** die Länge G₁ des ersten gekrümmten Abschnitts (4) im Bereich von 0,2 bis 0,4 L liegt.

18. Marknagel (1) nach einem der Ansprüche 1 bis 17, **dadurch gekennzeichnet, dass** der Krümmungsradius R₂ des zweiten gekrümmten Abschnitts (13) im Bereich von 10 bis 50 mm liegt.

## Claims

1. Intramedullary nail (1), especially for the tibia, with a proximal end part (2), a distal end part (3), suitable for introduction into the medullary space, and a central axis (6), which
A) has a total length L, ranging from 200 to 500 mm,
B) a first curved section (4) of length G₁ ≤ L and with the radius of curvature R₁ and
C) between the proximal end part (2) and the first curved section (4), a second curved section (13) of length G₂ ≤ G₁ and with a radius of curvature R₂ < R₁, **characterized in that**
D) the tangents at the two end points of a central section which includes the two curved sections (4, 13), enclose an angle gamma between 9° and 12°.

2. The intramedullary nail (1) of claim 1, **characterized in that** the length G₃ of the proximal end part (2) ranges from 1/6 L to 1/3 L.

3. The intramedullary nail (1) of claims 1 or 2, **characterized in that** the tangents t_{5,4} and t_{4,13} at the two end points of the first curved section (4) enclose an angle alpha ranging from 7° to 10° and preferably from 8° to 9°.

4. The intramedullary nail (1) of one of the claims 1 to 3, **characterized in that** the tangents t₄,₁₃ and t_{13,2} at the two end points of the second curved section (13) enclose an angle beta ranging from 1° to 3°.

5. The intramedullary nail (1) of one of the claims 1 to 4, **characterized in that** the proximal end part (2) axially adjoins the second curved section (13) and is constructed as a straight section (8) of length G₃ ≤ L.

6. The intramedullary nail (1) of one of the claims 1 to 5, **characterized in that** the first curved section (4) of length G₁ has a radius of curvature R₁ ranging from 300 to 1300 mm.

7. The intramedullary nail (1) of one of the claims 1 to 6, that the ratio L/R₁ ranges from 0,2 to 0,8.

8. The intramedullary nail (1) of one of the claims 1 to 7, **characterized in that** the distal end part (3) is constructed as a straight section (5) with the length "I" ≤ L.

9. The intramedullary nail (1) of claim 8, **characterized in that** the length "I" ranges from 0,20 L to 0,55 L.

10. The intramedullary nail (1) of claim 8, **characterized in that** the length "I" ranges from 0,25 L to 0,50 L.

11. The intramedullary nail (1) of one of the claims 1 to 10, **characterized in that** it has a longitudinal borehole (7), which is coaxial with the central axis (6).

12. The intramedullary nail (1) of one of the claims 1 to 11, **characterized in that**, in the region of the proximal end part (2), there is at least one locking hole (9) extending transversely to the central axis (6).

13. The intramedullary nail (1) of one of the claims 1 to 12, **characterized in that**, in the region of the distal end part (3), there is at least one locking hole (10) extending transversely to the central axis (6).

14. The intramedullary nail (1) of one of the claims 1 to 13, **characterized in that**, in the region of the distal end part (3), there are at least two locking holes (10) extending transversely to the central axis (6).

15. The intramedullary nail (1) of claim 14, **characterized in that** the two locking holes (10), extending transversely to the central axis, enclose an angle of 45° to 90° with one another.

16. The intramedullary nail (1) of one of the claims 1 to 15, **characterized in that** the distal end part (3) has three locking holes (10, 12, 10), the distance between the central locking hole (12) and the other two locking holes (10) being different.

17. The intramedullary nail (1) of one of the claims 1 to 16, **characterized in that** the length G₁ of the first curved section (4) ranges from 0,2 to 0,4 L.

18. The intramedullary nail (1) of one of the claims 1 to 17, **characterized in that** the radius of curvature R₂ of the second curved section (13) ranges from 10 to 50 mm.

## Revendications

1. Clou médullaire (1), en particulier pour le tibia, comprenant une partie d'extrémité proximale (2), une partie d'extrémité distale (3) appropriée pour l'introduction dans l'espace médullaire, et un axe central (6), qui
A) présente une longueur totale L se situant dans la plage allant de 200 mm à 500 mm ;
B) présente une première partie incurvée (4) de la longueur G₁ ≤ L et ayant le rayon de courbure R₁ ; et
C) présente une deuxième partie incurvée (13) - disposée entre la partie d'extrémité proximale (2) et la première partie incurvée (4) - de la longueur G₂ ≤ G₁ et ayant le rayon de courbure R₂ < R₁ ;
**caractérisé en ce que**
D) les tangentes forment dans les deux points d'extrémité d'une partie centrale englobant les deux parties incurvées (4 ; 13), un angle gamma compris entre 9° et 12°.

2. Clou médullaire (1) selon la revendication 1, **caractérisé en ce que** la longueur G₃ de la partie d'extrémité proximale (2) se situe dans la plage allant de 1/6 à 1/3 de L.

3. Clou médullaire (1) selon la revendication 1 ou 2, **caractérisé en ce que** les tangentes t_{5,4} et t_{4,13} forment dans les deux points d'extrémité de la première partie incurvée (4), un angle alpha se situant dans la plage allant de 7° à 10°, de préférence de 8° à 9°.

4. Clou médullaire (1) selon l'une quelconque des revendications 1 à 3, **caractérisé en ce que** les tangentes t_{4,13} et Q_{3,2} forment dans les deux points d'extrémité de la deuxième partie incurvée (13), un angle bêta se situant dans la plage allant de 1° à 3°.

5. Clou médullaire (1) selon l'une quelconque des revendications 1 à 4, **caractérisé en ce que** la partie d'extrémité proximale (2) est configurée en étant contiguë axialement à la deuxième partie incurvée (13) et comme partie droite (8) de la longueur G₃ ≤ L.

6. Clou médullaire (1) selon l'une quelconque des revendications 1 à 5, **caractérisé en ce que** la première partie incurvée (4) de la longueur G₁ présente un rayon de courbure R₁ se situant dans la plage allant de 300 mm à 1300 mm.

7. Clou médullaire (1) selon l'une quelconque des revendications 1 à 6, **caractérisé en ce que** le rapport L/R₁ se situe dans la plage allant de 0,2 à 0,8.

8. Clou médullaire (1) selon l'une quelconque des revendications 1 à 7, **caractérisé en ce que** la partie d'extrémité distale (3) est configurée comme une partie droite (5) dont la longueur "I" est inférieure ou égale à L.

9. Clou médullaire (1) selon la revendication 8, **caractérisé en ce que** la longueur "I" se situe dans la plage allant de 0,20 à 0,55 de L.

10. Clou médullaire (1) selon la revendication 8, **caractérisé en ce que** la longueur "I" se situe dans la plage allant de 0,25 à 0,50 de L.

11. Clou médullaire (1) selon l'une quelconque des revendications 1 à 10, **caractérisé en ce qu'**il présente un perçage longitudinal (7) coaxial par rapport à l'axe central (6).

12. Clou médullaire (1) selon l'une quelconque des revendications 1 à 11, **caractérisé en ce qu'**au moins un trou de verrouillage (9) s'étendant de façon transversale par rapport à l'axe central (6) est présent dans la zone de la partie d'extrémité proximale (2).

13. Clou médullaire (1) selon l'une quelconque des revendications 1 à 12, **caractérisé en ce qu'**au moins un trou de verrouillage (10) s'étendant de façon transversale par rapport à l'axe central (6) est présent dans la zone de la partie d'extrémité distale (3).

14. Clou médullaire (1) selon l'une quelconque des revendications 1 à 13, **caractérisé en ce que** deux trous de verrouillage (10) s'étendant de façon transversale par rapport à l'axe central (6) sont présents dans la zone de la partie d'extrémité distale (3).

15. Clou médullaire (1) selon la revendication 14, **caractérisé en ce que** les deux trous de verrouillage (10) s'étendant de façon transversale par rapport à l'axe central (6) forment entre eux un angle de 45° à 90°.

16. Clou médullaire (1) selon l'une quelconque des revendications 1 à 15, **caractérisé en ce que** la partie d'extrémité distale (3) présente trois trous de verrouillage (10, 12, 10), le trou de verrouillage central (12) présentant une distance variable par rapport aux deux autres trous de verrouillage (10).

17. Clou médullaire (1) selon l'une quelconque des revendications 1 à 16, **caractérisé en ce que** la longueur G₁ de la première partie incurvée (4) se situe dans la plage allant de 0,2 à 0,4 de L.

18. Clou médullaire (1) selon l'une quelconque des revendications 1 à 17, **caractérisé en ce que** le rayon de courbure R₂ de la deuxième partie incurvée (13) se situe dans la plage allant de 10 mm à 50 mm.
